(19)

**Europäisches Patentamt**
**European Patent Office**
**Office européen des brevets**

(11) **EP 3 581 091 A1**

(12) **EUROPEAN PATENT APPLICATION**

(43) Date of publication:
18.12.2019 Bulletin 2019/51

(21) Application number: 18177303.7

(22) Date of filing: 12.06.2018

(51) Int Cl.:
*A61B 5/00* (2006.01)  *A61B 5/024* (2006.01)
*A61B 5/145* (2006.01)  *A61B 5/1455* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventor: **DE HAAN, Gerard**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **SYSTEM AND METHOD FOR DETERMINING AT LEAST ONE VITAL SIGN OF A SUBJECT**

(57) The present invention relates to a system and method for determining at least one vital sign of a subject. To improve the ambient light robustness, said system comprises an illumination unit (2) configured to illuminate a skin region of the subject, said illumination unit comprising a first radiation source (20) and a second radiation source (21), which are configured to emit differently modulated electromagnetic radiation in two or more wavelength channels and/or in two or more polarization channels, a detection unit (4) configured to detect electromagnetic radiation reflected from the skin region of the subject and to generate detection signals, a processing unit (11) configured to demodulate the detection signals corresponding to the modulation applied by the illumination unit to obtain demodulated detection signals, and a vital signs determination unit (5) configured to determine, per wavelength or polarization channel, a statistical measure from said demodulated detection signals and to extract a vital sign from said statistical measures.

FIG.1

**Description**

FIELD OF THE INVENTION

The present invention relates to a system and method for determining at least one vital sign of a subject.

BACKGROUND OF THE INVENTION

Vital signs of a person, for example the heart rate (HR), the respiration rate (RR) or the (peripheral or pulsatile) blood oxygen saturation (SpO2; it provides an estimate of the arterial blood oxygen saturation SaO2), serve as indicators of the current state of a person and as powerful predictors of serious medical events. For this reason, vital signs are extensively monitored in inpatient and outpatient care settings, at home or in further health, leisure and fitness settings.
One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.
Photoplethysmography (PPG) is an optical measurement technique that evaluates a time-variant change of light reflectance or transmission of an area or volume of interest. PPG is based on the principle that the blood absorbs light more than the surrounding tissue, so variations in blood volume with every heartbeat affect the transmission or reflectance correspondingly. Besides information about the pulse rate (heart rate), a PPG waveform (also called PPG signal) can comprise information attributable to further physiological phenomena such as the respiration. By evaluating the transmittance and/or reflectance at different wavelengths (typically red and infrared), the blood oxygen saturation can be determined.
Conventional pulse oximeters (also called contact PPG device herein) for measuring the pulse rate and the (arterial) blood oxygen saturation of a subject are attached to the skin of the subject, for instance to a fingertip, earlobe or forehead. Therefore, they are referred to as 'contact' PPG devices. Although contact PPG is basically regarded as a non-invasive technique, contact PPG measurement is often experienced as being unpleasant and obtrusive, since the pulse oximeter is directly attached to the subject and the cables limit the freedom to move and might hinder a workflow.
Non-contact, remote PPG (rPPG) devices (also called camera-based devices or video health monitoring devices) for unobtrusive measurements have been proposed in the last decade. Remote PPG utilizes light sources or, in general, radiation sources, disposed at a distance from the subject of interest. Similarly, a detector, e.g. a camera or a photodetector, can be disposed at a distance from the subject of interest. Therefore, remote photoplethysmographic systems and devices are considered unobtrusive and well suited for medical as well as non-medical everyday applications.
Using the PPG technology, vital signs can be measured. Vital signs are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or strict accuracy requirements, where an improved robustness and accuracy of the vital sign measurement devices and methods is required, particularly for the more critical healthcare applications.
Video Health Monitoring (to monitor or detect e.g. heart rate, respiration rate, SpO2, actigraphy, delirium, etc.) is a promising emerging field. Its inherent unobtrusiveness has distinct advantages for patients with fragile skin, or in need of long-term vital signs monitoring, such as NICU patients, patients with extensive burns, mentally-ill patients that remove contact-sensors, or COPD patients who have to be monitored at home during sleep. In other settings such as in a general ward or emergency room, the comfort of contactless monitoring is still an attractive feature.
The measurement of one of the important vital signs, the blood oxygen saturation (SpO2), has recently shown to be feasible with camera-based rPPG. SpO2 measurements require an accurate detection of relative pulsatilities, i.e. the normalized amplitude of the pulsatile signal (AC/DC) in different wavelength channels. There are two major threats to the accuracy in remote measurement. The first is that the pulsatilities are low compared to the noise, while the second is that ballistocardiographic (BCG) motion modifies the relative pulsatilities in each channel.
Next to oxygen saturation, the saturation of other arterial blood gasses and blood species, e.g. HBCO, MetHB, HBCO2, bilirubin, may be obtained using the same (somewhat adapted) technique, and suffer from the same threat that the current invention aims to solve. The arterial blood components are selected with this technique because only the arterial blood is assumed to pulsate at the rhythm of the cardiac activity. It is hypothesized that, similarly, the respiratory cycle induces volume variations in the venous system, implying that the relative strength of the pulsations at the respiratory rhythm in different wavelengths may be used to analyze the venous blood components, e.g. to estimate the venous oxygen saturation (SvO2).
While being a promising new field, many challenges have to be overcome. A weakness of remote PPG systems is that their robustness often suffers when strong ambient light interferes with the intended irradiation of the skin for remote PPG, while skin segmentation is often hard, and multi spectral cameras expensive.

Hence, there is a need for an improved system and method for determining at least one vital sign of a subject to obtain results with higher reliability and robustness even in case of ambient illumination or interference.

SUMMARY OF THE INVENTION

It is an object of the present invention to provide a system and method for determining at least one vital sign of a subject, by which results with higher reliability and robustness, even in case of ambient illumination or interference, can be achieved.

In an aspect of the present invention a system is presented comprising

- an illumination unit configured to illuminate a skin region of the subject, said illumination unit comprising a first radiation source and a second radiation source, which are configured to emit differently modulated electromagnetic radiation in two or more wavelength channels and/or in two or more polarization channels,
- a detection unit configured to detect electromagnetic radiation reflected from the skin region of the subject and to generate detection signals,
- a processing unit configured to demodulate the detection signals corresponding to the modulation applied by the illumination unit to obtain demodulated detection signals, and
- a vital signs determination unit configured to determine, per wavelength or polarization channel, a statistical measure from said demodulated detection signals and to extract a vital sign from said statistical measures.

In a further aspect of the present invention, there is provided a corresponding method.

Preferred embodiments of the invention are defined in the dependent claims. It shall be understood that the claimed method has similar and/or identical preferred embodiments as the claimed system, in particular as defined in the dependent claims and as disclosed herein.

A weakness of remote PPG systems is that cameras, as conventionally used for detecting electromagnetic radiation reflected from (or transmitted through) the skin region of the subject have relatively low temporal sampling frequencies (i.e. picture rates), which prohibit low-cost (frequency/phase-multiplex) multi-wavelength solutions that can be very robust to ambient illumination. The robustness using HF-modulated light comes from the fact that the ambient illumination spectrum usually does not exhibit many high frequencies. Using high frequency modulation of the light sources, and demodulating the signal from the camera, makes the system virtually blind for ambient light variations. This can only work if the camera allows the high sampling rate required to capture the HF-modulated light, which severely limits this technique with the current state of camera technology.

Another problem with cameras to deal with in relation to ambient light is that they have a very limited dynamic rage, typically using 8, or maximally 12 bit brightness quantization. This implies that, even if frequency modulation is used, the modulated light can get lost through clipping of the optical sensor, if the additional, non-modulated ambient light is stronger than the modulated light, which may easily happen with sun-light.

A further concern with the use of cameras relates to privacy, an issue that can also be solved with the present invention.

The proposed system for remote PPG can effectively eliminate one or more of these weaknesses by choosing a detection unit for detecting electromagnetic radiation and for generating detection signals, from which statistics can be derived that allow dealing with the inherent pollution of the skin region with light reflection from non-skin surfaces. Preferably, a low-resolution 2D sensor, e.g. built as a small array of photo-diodes, is used so that very high sampling rates and a large dynamic range become feasible. This allows HF modulated radiation sources and can offer, combined with its very large dynamic range, a strong ambient light robustness.

The illumination unit comprises two radiation sources for emitting differently modulated electromagnetic radiation in two or more wavelength channels and/or in two or more polarization channels. The illumination unit may e.g. be realized by individual radiation (e.g. light) emitting elements, such as LEDs.

Each detection signal is hence (separately) demodulated twice (corresponding to the modulation applied by the first and second radiation sources), i.e. two demodulated detection signals are obtained per measured detection signal. In other words, every pixel of the detection unit is demodulated with the different modulation frequencies to obtain a pixel-value for each wavelength or polarization channel.

The vital sign is extracted from the statistical measures. As an example, if there are e.g. 16 photodiodes in the detection unit, each generating a detection signal, and if there are three wavelength channels. Preferably, from said detection signals at least two statistical measures are generated per wavelength channel, i.e. six statistical measures in total are derived. In an embodiment, a central tendency metric (e.g. mean or median of all pixels for each wavelength) and a dispersion metric (e.g. the variance of all pixels for each wavelength) are obtained per wavelength channel. The vital signs then are extracted from the six statistical measures (two metrics per three wavelength channels).

As used herein, a statistical measure (also called statistical parameter value) can refer to a measure indicative of values of pixels of the detection unit. Statistical dispersion (also called variability, scatter, or spread) can be indicative of an extent to which a distribution is stretched or squeezed. Common examples of measures of statistical dispersion are the variance, standard deviation, and interquartile range. A candidate signal can be determined by concatenating statistical measures of the corresponding detection signals over time. For example, at each instant of time a standard deviation value of the pixel values at this instant of time is determined and the subsequent standard deviation values form the candidate signal over time.

Optionally, additional steps such as normalizing a candidate signal, e.g. by dividing it by its (moving window) temporal mean, taking its logarithm and/or removing an offset, may be performed. Moreover, (pre-) processing steps such as weighting pixels by a weighting map, resizing, rescaling, resampling or cropping of the (weighted) detection signals. A (weighted) detection signal as used herein may thus also refer to such a (pre-) processed (weighted) detection signal.

It shall be noted here that the terms "light" and "radiation" as used herein shall generally be understood as meaning the same, in particular electromagnetic radiation in the visible and infrared spectrum. Preferably, radiation in the range from 400 nm to 1000 nm is used according to the present invention.

According to an embodiment, said statistical measure is indicative of at least one of a standard deviation, a variance, mean absolute difference, median absolute difference and/or an interquartile range. Hence, a statistical measure indicative of a statistical dispersion is evaluated instead of the conventional approach of averaging pixels (i.e. evaluating a central tendency metric) in a region-of-interest.

According to an embodiment, the first and second radiation sources are configured to emit electromagnetic radiation at different wavelengths or wavelength ranges or mixtures of wavelengths. The (pseudo)-color signals can then be demodulated robustly from these modulated wavelength channels.

In another embodiment, the first and second radiation sources are configured to emit electromagnetic radiation with different polarization. It is also possible to implement both options in an embodiment. This may further increase ambient light robustness and/or decrease sensitivity for specular reflections.

Optionally, the illumination unit is configured to apply modulation frequencies for modulating the emitted electromagnetic radiation in the range higher than 1 kHz, in particular in a frequency range from 10 kHz to 100 MHz. This enables to clearly distinguish them from ambient illumination variations. In offices current fluorescent lamps emit 100/120 Hz with potential harmonics, while more recent LEDs often emit strong frequencies in the range up to a kHz. Some dimmable fluorescent lamps may even emit frequencies as high as 20 kHz. Hence, quite high frequencies are preferred to prevent all imaginable interferences. These frequencies are clearly unobtainable with the current camera technology. Hence, this embodiment provides for a good robustness against ambient light variations.

The first radiation source maybe configured to apply a first modulation frequency for modulating the emitted electromagnetic radiation, which is at least 8 Hz apart from a second modulation frequency applied by the second radiation source for modulating the emitted electromagnetic radiation. This enables a good separation of the wavelength channels and further contributes to improving the robustness against ambient light.

The detection unit preferably comprises a plurality of detection elements, in particular an array of photo diodes, a CCD array or a CMOS array single photo-diode or an array of photo-diodes, which represents an inexpensive but effective solution. For instance, a relatively small array of photo-diodes may be used, i.e. an array that is just large enough to capture individual polarization directions or that is equipped with individual optical filters to further improve ambient light robustness.

The system may further comprise a polarization unit configured to apply a polarization to the electromagnetic radiation reflected from the subject's skin region. For instance, a single (common) polarizer may be arranged in front of all pixels of the detection unit. After demodulation, both the cross- and the parallel polarized channels can be obtained. In another implementation some detection elements may have a polarizer identical to the first polarization of the first radiation source and the other detection elements may have a polarizer identical to the second polarization of the second radiation source.

The system may further comprise an optical filter unit configured to filter the electromagnetic radiation reflected from the subject's skin region to allow only modulation frequencies used by the illumination unit to pass. In a cost-effective embodiment a common filter is provided that only transmits the wavelengths used in the modulated radiation sources. This makes the sensor "blind" for all other wavelengths and hence increases ambient light robustness (typically ambient light has a very broad spectrum (e.g. sunlight)). In another implementation individual pixels may be provided with filters that pass less than all used wavelengths (e.g. only one or two wavelengths in case 3 different wavelengths are emitted by the radiation sources). This reduces, however, the effective number of pixels per wavelength, but further increases the ambient light robustness. Even in case the number of different filters used equals the number of wavelengths (e.g. three LEDs and three groups of pixels with different matched filters), the modulation helps, since most variations in a wavelength-interval (e.g. due to motion) may not fall in the narrow band around the modulation frequency.

The processing unit may further be configured to apply synchronous demodulation on the detection signals to obtain the demodulated detection signals.

The extraction of a physiological parameter, e.g. a vital sign, of the subject based on the candidate signal can be performed using known techniques. Exemplary techniques include but are not limited to evaluating a fixed weighted sum over candidate signals of different wavelength channels (RGB, IR), blind source separation techniques advantageously involving both candidate signals such as blind source separation based on selecting the most pulsatile independent signal, principal component analysis (PCA), independent component analysis (ICA), CHROM (chrominance-based pulse extraction), POS (wherein the pulse is extracted in a plane orthogonal to a skin-tone vector), PBV method (which uses a predetermined signature of a blood volume pulse vector), or APBV (an adaptive version of the PBV-method, also allowing blood oxygen saturation monitoring). It should be noticed that optional pre-processing may be applied to the candidate signals, such as e.g. correction of gains of one or more channels, to further improve the performance.

Hence, in an embodiment the vital sign determination unit is configured to determine a vital sign from the demodulated detection signals by linearly combining the demodulated detection signals by a weighted combination, wherein weights of said weighted combination are determined by blind signal separation, in particular by principal component analysis or independent component analysis, and by selecting a component channel of the combined detection signals according to a predetermined criterion.

In another embodiment the vital sign determination unit is configured to determine a vital sign from the demodulated detection signals by linearly combining the demodulated detection signals by a weighted combination using weights resulting in a pulse signal for which the products with the original detection signals equals the relative pulsatilities as represented by the respective signature vector, a signature vector providing an expected relative strength of the detection signal in the original detection signals.

At least one of the first and second radiation sources is configured to emit a controllable narrow radiation beam. For this purpose, a control unit may be provided. One or more of the direction, the beam width, and the strength of a radiation beam emitted by a radiation source may hence be controlled. This may be used e.g. to illuminate primarily, or only, skin portions of the subject and leave other portions of the subject, or her/his environment, non-illuminated.

Most effectively, the illumination unit is configured to illuminate the skin region of the subject with electromagnetic radiation within the wavelength range from 300 nm to 1000 nm.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a first embodiment of a system according to the present invention;
Fig. 2 shows a schematic diagram of a second embodiment of a system according to the present invention;
Fig. 3 shows a schematic diagram of a third embodiment of a system according to the present invention; and
Fig. 4 shows a schematic diagram of a fourth embodiment of a system according to the present invention.

DETAILED DESCRIPTION OF THE INVENTION

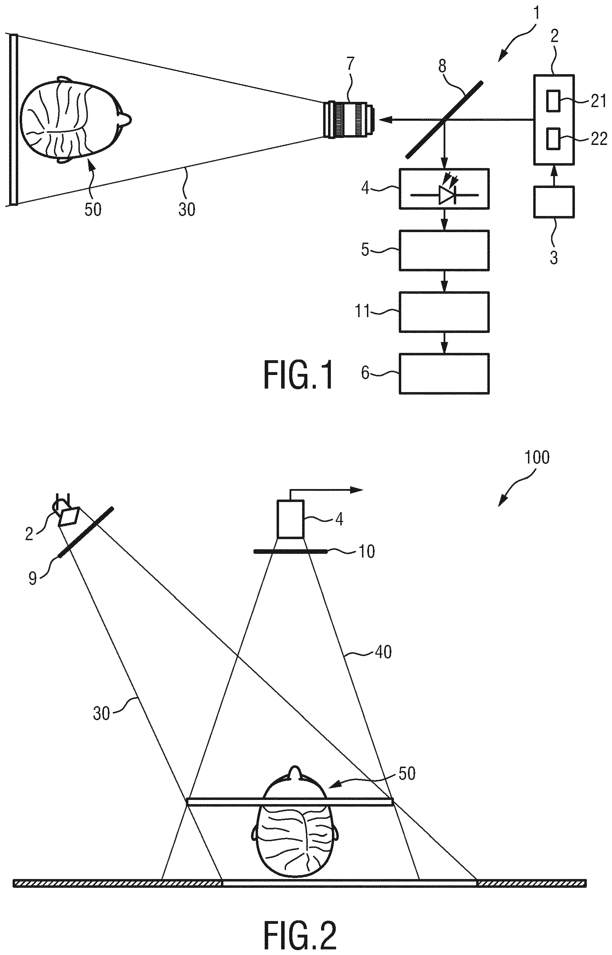

Fig. 1 shows a schematic diagram of a first embodiment of a system 1 for determining at least one vital sign of a subject 50 according to the present invention. The subject 50 may be a patient, e.g. lying in a bed in a hospital or other healthcare facility, but may also be a neonate or premature infant, e.g. lying in an incubator, or person at home or in a different environment.

The system 1 comprises an illumination unit 2 configured to illuminate a skin region of the subject 50. The illumination unit 2 comprises a first radiation source 20 and a second radiation source 21, which are configured to emit differently modulated electromagnetic radiation in two or more wavelength channels and/or in two or more polarization channels. For instance, the radiation sources may be configured as LEDs. In embodiments, a plurality of radiation sources, i.e. more than two radiation sources, may be provided.

The radiation sources 20, 21 may be controllable. For this purpose, a control unit 3 configured to control the radiation sources 21, 22 of the illumination unit 2 may be provided, which may e.g. be a controller or processor.

The system 1 further comprises a detection unit 4 configured to detect electromagnetic radiation reflected from the skin region of the subject 50 and to generate detection signals from the detected electromagnetic radiation. The detection unit 4 may e.g. be a single photo-diode or an array of photo-diodes.

The system 1 further comprises a processing unit 11 configured to demodulate the detection signals corresponding to the modulation applied by the illumination unit 2 to obtain demodulated detection signals.

Finally, the system 1 comprises a vital signs determination unit 5 configured to determine, per wavelength or polarization channel, a statistical measure from said demodulated detection signals and to extract a vital sign from said statistical measures. The processing unit 11 and/or the vital signs determination unit 5 may e.g. be a processor or computer or dedicated hardware.

The system 1 may optionally further comprise an interface 6 for displaying the determined information and/or for providing medical personnel with an interface to change settings of the system's components. Such an interface may comprise different displays, buttons, touchscreens, keyboards or other human machine interface means.

If the radiation sources are e.g. LEDs then the modulation of the emitted electromagnetic radiation is most easily achieved by modulating the current through the LEDs. An electronic circuit may provide a current modulated around a bias value, e.g. in a sinusoidal fashion (although other waveforms are possible and may be attractive). For light sources other than LED, it may be difficult to achieve such a HF modulation with the source itself so that an optical modulator may additionally be used to achieve the modulation of the emitted electromagnetic radiation.

A system 1 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, a wearable, a handheld device like a smartphone, or the like. The uni- or bidirectional communication between two or more components of the system 1 may work via a wireless or wired communication interface.

The embodiment shown in Fig. 1 is a low-cost embodiment, particularly suitable for automotive applications. The illumination unit 2 and the detection unit 4 are viewing the scene with the subject 50 through a common optical element 7, e.g. a common lens. Since the face of the subject 50 is relatively close to the illumination unit 2 it reflects far more light than the surfaces behind the subject. A semi-transparent mirror 8 maybe used in such an exemplary embodiment, but may in practice not sufficiently suppress direct light to the detection unit 4, i.e. separate lenses for the illumination unit 2 and the detection unit 4 may instead be used.

The signals from the individual detection elements of the detection unit 4 are demodulated to retrieve the different wavelength / polarization channels per detection unit 4, preferably in a 2D array of detection elements. Next, the statistical measures are computed, e.g. central tendency (e.g. mean or median of all demodulated detector signals or channels from the array), and a dispersion (e.g. variance or standard deviation of all demodulated detector signals or channels from the array) metric for the individual wavelength / polarization channels to deal with the fact that (part of) the reflected light falling on some of the detection elements may be from non-skin surfaces (it should be noted that no Region of Interest (RoI) detector is generally used, but the whole set of detection signals representing the skin and some background (which could be considered a polluted RoI).

Finally, the vital sign is extracted from the statistical measures of the channels, preferably of all channels, e.g. a PPG signal (pulse) can be achieved as a linear combination of the statistical measures of all wavelength channels. Typically, any of the previously reported methods (PCA, ICA, PBV, CROM, POS, APBV, PBVGOP) can be applied on the individual statistical metrics, e.g. PCA on the mean, and PCA on the variance, leading to two PPG signals from which the output PPG signal is obtained by selection or combination, e.g. linear weighting using a quality indicator (e.g. skewness of the spectrum, height of the highest peak in the normalized spectrum, etc.). The background for this choice is that the mean works very well in case few channels correspond to non-skin surfaces, while the variance works better in case a significant part of the channels correspond to non-skin and possibly only a few channels correspond to skin.

Typically, the electromagnetic radiation is in the range of 400 nm to 1000 nm for pulse, respiration and blood oxygen saturation measurement, particularly in the range of 620 nm to 920 nm. This particular range is most suitable for SpO2 measurement and is attractive for unobtrusive monitoring during sleep (for near darkness further limitation to a range of 760 nm to 920 nm may be preferred), but the visible part of the spectrum may allow a higher quality in case visibility of the light is not obtrusive (i.e. NIR is not necessarily the preferred option in all cases). The detection signals may be acquired by a photo-sensor (or a photo-sensor array) remotely sensing the subject's skin, i.e. at least some skin needs to be imaged by the detector, but the detector may see some background as well.

By use of the illumination unit 2, illumination of non-skin surfaces can in principle be prevented or minimized. Various methods can be used to realize this, but an example is that the illumination is embedded in a feedback loop, where the quality (e.g. SNR) of the extracted PPG signal is optimized by varying the intensity of "pixels" of the illumination unit.

In practical embodiments, the illumination unit 2 may emit radiation in at least two or three different wavelength intervals (RGB, or invisible wavelengths using NIR) in a modulated (frequency or phase) fashion to enable ambient light robustness. The (pseudo)-color signals can then be demodulated robustly from these modulated wavelength channels (or alternatively be obtained from different photo-diodes equipped with different optical filters in which case the modulation only serves the increased robustness for ambient light and can be identical (i.e. same modulation frequency) for all wavelengths), and the PPG signal can be extracted from the wavelength channels motion-robustly using the same basic algorithms that have been proposed for camera-based extraction (such as ICA, PCA, CHROM, POS, (A)PBV-method, etc.), as will be explained in more detail below.

To achieve a good robustness for ambient illumination variations, the illumination unit 2 can be modulated at a relatively high frequency, e.g. between 1 kHz and several MHz, where the ambient light spectrum typically can be expected to be fairly clean, and thus cause no interference with the demodulated wavelength channels. Because the detector is a high-speed detector, e.g. a photo-diode, rather than the imaging sensor typically used in remote PPG applications, these high frequencies are easily feasible. Different wavelengths/polarizations can be modulated at different frequencies (in case of monochrome detector(s)), although the channels need not be separated very far. Around 8 Hz separation could already suffice, as the sidebands due to the pulse signal is limited to about +/- 4 Hz for the maximum human pulse rate.

In another embodiment ambient light robustness is improved by using a detection unit, e.g. a photo-detector, equipped with an optical filter that selectively transmits the wavelengths used in the modulated illumination unit, but blocks all (or most) other ambient light, e.g. by means of a visible light-blocking filter.

A further embodiment may increase ambient light robustness by using separate photo-detectors for each wavelength used, where each photo-detector maybe equipped with an illumination unit matching optical filter.

To improve ambient light robustness or decrease sensitivity for specular reflections, polarizers may be used in front of the detection unit and/or the illumination unit. Also having two signals obtained with different polarization filters may in itself allow for a combination of the (partly independent) signals that is cleaner than the individual signals. This enables options with a single wavelength, or provides two times more detection signals to be combined than without polarizers.

The control unit 3 for controlling the illumination unit 2 may be configured to control the direction and/or width and/or intensity of the (generally relatively narrow) beam 30 from the illumination unit 2 so that e.g. as much skin as possible and as little non-skin surfaces as possible are illuminated, using the same optimization criterion. Preferably, the radiation sources 21, 22 can be controlled individually or in groups to achieve this effect.

In the embodiment of the system 1 shown in Fig. 1 different wavelengths can be multiplexed or multiple LEDs (different wavelengths) modulated with different frequencies. Very high modulation frequencies (kHz - MHz) are possible, and a good separation is possible using e.g. a product detector (or heterodyne demodulator). For instance, demodulation may be effected by computing the inner-product over a sliding analysis window with a sine- and a cosine-wave, and computing the square root of the squared inner-products, and finally low-pass filtering (e.g. 4 Hz cut-off) the resulting values from the sliding window processing. The high modulation frequencies prevent problems with motion, and can make the system very robust for ambient illumination, i.e. the frequency selectivity makes the system "blind" for ambient illumination variations, which are typically lower than 1 kHz (50/100 Hz for classical light sources, several hundreds of Hz for LED light), and lower than 100 kHz. In this case, the relative proximity of the face (compared to background) can prevent the necessity for pixelated light, or alternatively a highly absorbing background immediately behind the subject can prevent damaging contributions from non-skin surfaces.

Fig. 2 shows a schematic diagram of a further embodiment of a system 100 according to the present invention. Mainly the illumination unit and the detection unit are illustrated, while other components of the system like the control unit and the vital signs detection unit are omitted.

In this embodiment of the system 100, it is possible to have the illumination unit 2 cast a narrow beam 30 towards the subject 50, e.g. a beam 30 that is wide enough to only allow the necessary freedom to move as a car driver, and have the detection unit 4 view the subject 50 with a similarly narrow viewing angle 40. Hence, different from the system 1, the light path of source and sensor are separate in this embodiment. The face is illuminated most because it is closest to the illumination unit 2, hence the background has little effect on the signal quality (or highly absorbing background (e.g. headrest)). Thus, the skin/face contributes most to the light on the detector array (significantly), but the use of the one or more statistical metrics allows vital sign extraction, even when there is pollution of the detected light from non-skin surfaces.

Both light paths 30 and 40 are narrow and, provided their intersection mainly contains skin, the PPG signal will be of good quality even if the background is not black. Using a dark background may be a viable option in some applications too. If the background plays a significant part in the reflected light from the scene, it is still possible to get a high quality PPG signal by combining the signals formed by the one or more statistical metrics (e.g. central tendency and dispersion).

Using a photo-diode as detection unit, its detection signal can be synchronously demodulated in the kHz-range. For instance, a 3-wavelength system (e.g. using 760 nm, 800 nm and 900 nm) modulated in frequency multiplex, can have it central frequencies around 10 kHz with the wavelength channels being at least 8 Hz apart, e.g. 10, 11, or 12 kHz). This separation is preferred due to the range of possible pulse frequencies (0.5-4 Hz, which gives +/- 4 Hz side-bands to the modulation frequencies). Therefore, they should be 8 Hz apart to prevent interference.

There are other interference considerations though. Non-linearities in the radiation source (e.g. LEDs) cause harmonics of the modulation frequencies, which also should not cause interference in the pulse band of other wavelengths (e.g. a 10 kHz modulation frequency causes harmonics at 20, 30, 40, etc. kHz). If any of the demodulators mixes down any of these harmonics into the pulse rate band interference results (e.g. when using 10 kHz for wavelength 1, other modulation frequencies should also stay clear of 40 kHz +/-32 Hz to prevent interference).

Hence, there may be various constraints to choosing the modulation frequencies. There is not only a lower bound, but there are many frequency bands which should not be used, as becomes clear from the above example.

It is also possible to modulate the received light with different polarization directions (cross and parallel to the polarization of the light of the illumination beam 20). For this purpose polarizers 9 and 10 may be used. The use of polarized light for illumination and a cross-polarizer 10 in front of or as part of the detection unit 4 (in particular in front of the sensor that senses the received radiation) may help suppress specularly reflected light. Also, the use of polarizers may (partially) replace the use of extra wavelengths to improve motion robustness and the SNR of the PPG signal.

In order to suppress specular reflection it suffices to use a single polarizer for all radiation sources and an orthogonal (cross) polarizer in front of the detection unit. To improve motion robustness, multiple options exists:

   a) use a single polarizer in front of all radiation sources and a cross polarizer and a parallel polarizer in front of two detection elements (forming the detection unit);
   b) alternate (switch) the polarization in front of a single detection element (forming the detection unit);
   c) use a single polarizer in front of the detection unit and switch the polarization in front of the radiation sources; or
   d) use multiple polarizers on parallel radiation sources.

Generally, as polarizer a polarization filter may be used. However, for this purpose, not only transmission filters can be employed, but reflectors and/or mirrors (e.g. polarization mirrors) maybe used to achieve the same effect.

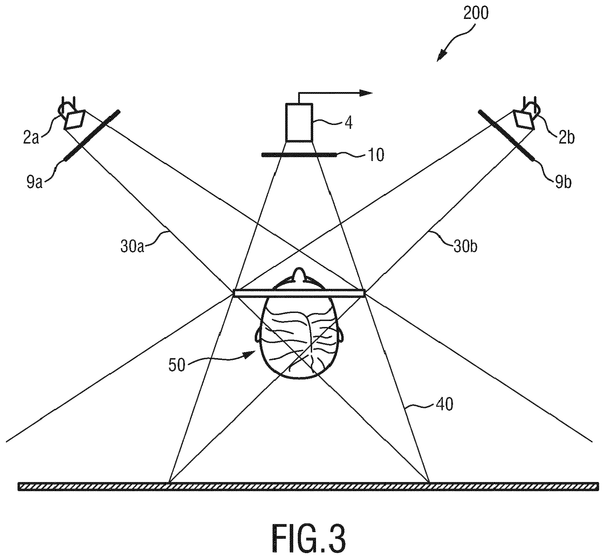

Fig. 3 shows a schematic diagram of a third embodiment of a system 200 according to the present invention. In this embodiment multiple illumination units 2a, 2b are used each with a relatively narrow beam 30a, 30b and optionally each with a polarizer 9a, 9b. Only objects in the intersection of the narrow beams 30a, 30b with the narrow sensor viewing angle 40 will contribute to the detection signal.

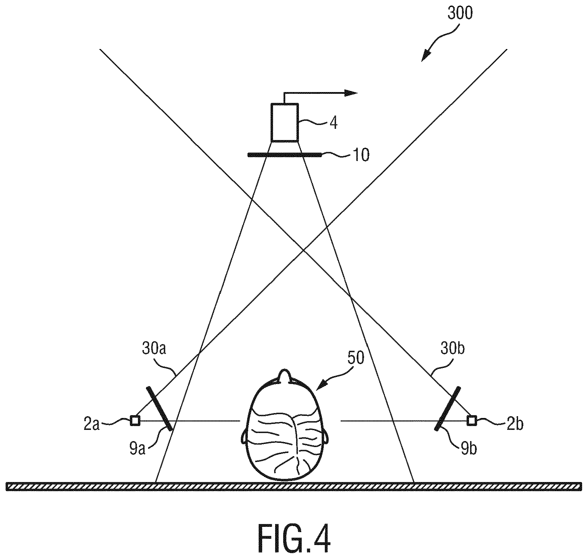

Fig. 4 shows a schematic diagram of a fourth embodiment of a system 300 according to the present invention. This embodiment is particularly suitable for automotive application since background illumination can be prevented altogether. The subject's face is illuminated from the sides, preferably with polarized light. The detection unit 4 is equipped with a cross-polarizer 10 and consequently only sees reflections that have been depolarized by translucent objects in its view. In the car it should be relatively easy to prevent any other (significantly reflecting) objects in the view, so that only the scattered light reflected from the skin is "seen" by the detection unit 4.

A further alternative embodiment employs a very narrow beam (narrow enough to cause an illuminated area that is smaller than the aimed for body-part (e.g. a face)) of light that can be directed by a controller. In this embodiment the detection unit may "see" a larger environment, but it can nonetheless give a good quality PPG signal if the spot is reaching the skin of a subject only. Possibly, the controller changes the orientation of the light beam, in this way optimizing the PPG signal quality.

Generally, a PPG signal results from variations of the blood volume in the skin. Hence, the variations give a characteristic pulsatility "signature" when viewed in different spectral components of the reflected/transmitted light. This "signature" is basically resulting as the contrast (difference) of the absorption spectra of the blood and that of the blood-less skin tissue. If the detector, e.g. a camera or sensor, has a discrete number of color channels, each sensing a particular part of the light spectrum, then the relative pulsatilities in these channels can be arranged in a "signature vector", also referred to as the "normalized blood-volume vector", PBV. It has been shown in G. de Haan and A. van Leest, "Improved motion robustness of remote-PPG by using the blood volume pulse signature", Physiol. Meas. 35 1913, 2014, which is herein incorporated by reference, that, if this signature vector is known, then a motion-robust pulse signal extraction on the basis of the color channels and the signature vector is possible. For the quality of the pulse signal, it is essential though that the signature vector is accurate, as otherwise the known methods mixes noise into the output pulse signal in order to achieve the prescribed correlation of the pulse vector with the normalized color channels as indicated by the signature vector.

Details of the PBV method and the use of the normalized blood volume vector (called "predetermined index element having a set orientation indicative of a reference physiological information") have also been described in US 2013/271591 A1, whose details are also herein incorporated by reference.

The characteristic wavelength-dependency of the PPG signal varies when the composition of the blood changes. Particularly, the oxygen saturation of the arterial blood has a strong effect on the light absorption in the wavelength range between 620nm and 780nm. This changing signature for different SpO2 values leads to relative PPG pulsatility that depends on the arterial blood oxygen saturation. This dependency can be used to realize a motion-robust remote SpO2 monitoring system that has been named adaptive PBV method (APBV) and is described in detail in M. van Gastel, S. Stuijk and G. de Haan, "New principle for measuring arterial blood oxygenation, enabling motion-robust remote monitoring", Nature Scientific Reports, Nov. 2016. The description of the details of the APBV method in this document is also herein incorporated by reference.

The PBV method gives the cleanest pulse signal when the PBV vector, reflecting the relative pulsatilities in the different wavelength channels is accurate. Since this vector depends on the actual SpO2 value, testing the PBV method with different PBV vectors, corresponding to a range of SpO2 values, the SpO2 value results as the one corresponding to the PBV vector giving the pulse-signal with the highest SNR.

In the following some basic considerations with respect to the PBV method shall be briefly explained, using RGB-wavelength channels as an example.

The beating of the heart causes pressure variations in the arteries as the heart pumps blood against the resistance of the vascular bed. Since the arteries are elastic, their diameter changes in synchrony with the pressure variations. These diameter changes occur not only in the big arteries, but even in the smaller vessels of the skin at the arterial side of the capillary bed, the arterioles, where the blood volume variations cause a changing absorption of the light.

The unit length normalized blood volume pulse vector (also called signature vector) is defined as PBV, providing the relative PPG-strength in the red, green and blue camera signal, i.e.

$$\vec{P}_{bv} = \frac{\left[\sigma(\vec{R}_n), \sigma(\vec{G}_n), \sigma(\vec{B}_n)\right]}{\sqrt{\sigma^2(\vec{R}_n) + \sigma^2(\vec{G}_n) + \sigma^2(\vec{B}_n)}}$$

with $\sigma$ indicating the standard deviation.

To quantify the expectations, the responses $H_{red}(w)$, $H_{green}(w)$ and $H_{blue}(w)$ of the red, green and blue channel, respectively, were measured as a function of the wavelength w, of a global-shutter color CCD camera, the skin reflectance of a subject, $\rho_s(w)$, and used an absolute PPG-amplitude curve PPG(w). From these curves, shown e.g. in Fig. 2 of the above cited paper of de Haan and van Leest, the blood volume pulse vector PBV is computed as:

$$\vec{P}_{bv}^T = \begin{bmatrix} \dfrac{\int\limits_{w=400}^{700} H_{red}(w)I(w)PPG(w)\,dw}{\int\limits_{w=400}^{700} H_{red}(w)I(w)\rho_s(w)\,dw} \\[3pt] \dfrac{\int\limits_{w=400}^{700} H_{green}(w)I(w)PPG(w)\,dw}{\int\limits_{w=400}^{700} H_{green}(w)I(w)\rho_s(w)\,dw} \\[3pt] \dfrac{\int\limits_{w=400}^{700} H_{blue}(w)I(w)PPG(w)\,dw}{\int\limits_{w=400}^{700} H_{blue}(w)I(w)\rho_s(w)\,dw} \end{bmatrix}$$

which, using a white halogen illumination spectrum I(w), leads to a normalized PBV = [0.27, 0.80, 0.54].

The blood volume pulse predicted by the used model corresponds reasonably well to an experimentally measured normalized blood volume pulse vector, PBV = [0.33, 0.78, 0.53] found after averaging measurements on a number of subjects under white illumination conditions. Given this result, it was concluded that the observed PPG-amplitude, particularly in the red, and to a smaller extent in the blue camera channel, can be largely explained by the crosstalk from wavelengths in the interval between 500 and 600 nm. The precise blood volume pulse vector depends on the color filters of the camera, the spectrum of the light and the skin-reflectance, as the model shows. In practice, the vector turns out to be remarkably stable though given a set of wavelength channels (the vector will be different in the infrared compared to RGB-based vector).

It has further been found that the relative reflectance of the skin, in the red, green and blue channel under white illumination does not depend much on the skin-type. This is likely because the absorption spectra of the blood-free skin is dominated by the melanin absorption. Although a higher melanin concentration can increase the absolute absorption considerably, the relative absorption in the different wavelengths remains the same. This implies an increase of melanin darkens the skin but hardly changes the normalized color of the skin. Consequently, also the normalized blood volume pulse PBV is quite stable under white illumination. In the infrared wavelengths, the influence of melanin is further reduced as its maximum absorption occurs for short wavelengths (UV-light) and decreases for longer wavelengths.

The main reason the PBV vector is not affected much by the melanin is that melanin is in the epidermis and therefore acts as an optical filter on both the AC and the DC. Hence, it may reduce the pulsatility, but at the same time also the DC value of the reflection. Hence the AC/DC (relative pulsatility) does not change at all.

The stable character of PBV can be used to distinguish color variations caused by blood volume change from variations due to alternative causes, i.e. the stable PBV can be used as a "signature" of blood volume change to distinguish their color variations. The known relative pulsatilities of the color channels PBV can thus be used to discriminate between the pulse-signal and distortions. The resulting pulse signal S using known methods can be written as a linear combination (representing one of several possible ways of "mixing") of the individual DC-free normalized color channels:

$$S = W\,C_n$$

with $WW^T = 1$ and where each of the three rows of the 3 x N matrix $C_n$ contains N samples of the DC-free normalized red, green and blue channel signals $R_n$, $G_n$ and $B_n$, respectively, i.e.:

$$\vec{R}_n = \frac{1}{\mu(\vec{R})}\vec{R} - 1, \quad \vec{G}_n = \frac{1}{\mu(\vec{G})}\vec{G} - 1, \quad \vec{B}_n = \frac{1}{\mu(\vec{B})}\vec{B} - 1.$$

Here the operator $\mu$ corresponds to the mean. Key difference between the different methods is in the calculation of the weighting vector W. In one method, the noise and the PPG signal may be separated into two independent signals built as a linear combination of two color channels. One combination approximated a clean PPG signal, the other contained noise due to motion. As an optimization criterion the energy in the pulse signal may be minimized. In another method a linear combination of the three color channels may be used to obtain the pulse signal.

The PBV method generally obtains the mixing coefficients using the blood volume pulse vector as basically described in US 2013/271591 A1 and the above cited paper of de Haan and van Leest. The best results are obtained if the band-passed filtered versions of $R_n$, $G_n$ and $B_n$ are used. According to this method the known direction of PBV is used to discriminate between the pulse signal and distortions. This not only removes the assumption (of earlier methods) that the pulse is the only periodic component in the video, but also eliminates assumptions on the orientation of the distortion signals. To this end, it is assumed as before that the pulse signal is built as a linear combination of normalized color signals. Since it is known that the relative amplitude of the pulse signal in the red, green and blue channel is given by PBV, the weights, $W_{PBV}$, are searched that give a pulse signal S, for which the correlation with the color channels $R_n$, $G_n$, and $B_n$ equals PBV

$$\vec{S}C_n^T = k\vec{P}_{bv} \Leftrightarrow \vec{W}_{PBV}C_nC_n^T = k\vec{P}_{bv}, \qquad (1)$$

and consequently the weights determining the mixing are determined by

$$\vec{W}_{PBV} = k\vec{P}_{bv}Q^{-1} \text{ with } Q = C_nC_n^T, \qquad (2)$$

and the scalar k is determined such that $W_{PBV}$ has unit length. It is concluded that the characteristic wavelength dependency of the PPG signal, as reflected in the normalized blood volume pulse, PBV, can be used to estimate the pulse signal from the time-sequential RGB pixel data averaged over the skin area. This algorithm is referred to as the PBV method.

In other words, the weights indicate how the detection signals should be (linearly) combined in order to extract a pulse signal from the detection signals. The weights are unknown and need to be computed/selected.

The signature vector (PBV vector) represent the given (known or expected) relative pulsatilities in different wavelength channels (i.e. the detection signals), caused by the absorption spectrum of the blood and the penetration of light into the skin (if photons are more absorbed by blood, a volume change of blood leads to a larger signal than when the blood is nearly transparent). With this knowledge, and the observed data (i.e. the detection signals) the weights (e.g. a weight vector) can be determined. The resulting weights are data dependent, i.e. depend on the detection signals.

Since the pulse signal has a different ratio AC/DC (this is also called the relative signal strength / pulsatility) in each wavelength channel, it can be seen that the spectrum shows the pulse peak in the spectrum with different peak values for the different colors. This spectrum is the result of a Fourier analysis, but it basically means that if a sinusoid having the pulse frequency is correlated (multiplied) with the detection signals (RGB in the example, NIR-wavelengths for SpO2), exactly the peak values in the spectrum are obtained, which by definition are called the signature vector (PBV vector): these peak values are the relative strength of the normalized amplitudes of the pulse signal in the different detection signals.

The consequence of this is that a clean pulse signal S can be obtained (assuming the pulse signal is the result of a weighted sum of the detection signals), using this prior knowledge (i.e. the signature vector). One option to do this is to compute an inversion of a covariance matrix Q of the normalized detection signals $C_n$. Hence, the weights W to linearly mix the detection signals in order to extract the pulse signal S can be computed from the covariance matrix of the detection signals in the current analysis window (Q, which is data dependent, i.e. changes continuously over time), using the constant signature vector PBV.

It is recognized that e.g. in the NIR-light spectrum, particularly between 620 and 770nm, the blood absorption spectrum changes depending on the SpO2 level. For this reason it is proposed to extract the pulse signal with different signature vectors (different PBV vectors), where each PBV vector is chosen to correspond with the relative pulsatilities in the detection signals for a particular vital sign value, e.g. an SpO2 value. Since the extracted pulse signal quality depends on the correctness of the PBV vector, the different extracted pulse signals will have a different quality. By selecting the best quality pulse signal, the vital sign value (e.g. SpO2 value) from the signature vector that caused this favorable pulse signal.

In another embodiment the APBV method is used to extract an SpO2 value from two or more different combinations of three wavelength channels, e.g. from [λ1, λ2], from [λ1, λ3], and/or from [λ1, λ2, λ3]. In the following some basic considerations with respect to the APBV method shall be briefly explained.

Instead of extracting features from the PPG waveforms, APBV determines SpO2 indirectly based on the signal quality of the pulse signals extracted with SpO2 'signatures'. This procedure can mathematically be described as:

$$SpO_2 = \underset{SpO_2 \in \mathbf{SpO_2}}{\mathrm{argmax}} \; SNR\left(\overbrace{k\vec{P}_{bv}(SpO_2)[\mathbf{C_n C_n^T}]^{-1}\mathbf{C_n}}^{\vec{W}_{PBV}}\right), \tag{3}$$

where $C_n$ contains the DC-normalized color variations and scalar k is chosen such that $\vec{W}_{PBV}$ has unit length. The SpO2 signatures compiled in $\overrightarrow{P_{bv}}$ can be derived from physiology and optics. Assuming identical cameras the PPG amplitudes of N cameras can be determined by:

$$\vec{P}_{bv} = \left\| \begin{bmatrix} \left(\frac{AC}{DC}\right)^1 \\ \left(\frac{AC}{DC}\right)^2 \\ \vdots \\ \left(\frac{AC}{DC}\right)^N \end{bmatrix} \right\| = \left\| \begin{bmatrix} \dfrac{\int_\lambda I(\lambda)F^1(\lambda)C(\lambda)PPG(\lambda)d\lambda}{\int_\lambda I(\lambda)F^1(\lambda)C(\lambda)\rho_s(\lambda)d\lambda} \\ \dfrac{\int_\lambda I(\lambda)F^2(\lambda)C(\lambda)PPG(\lambda)d\lambda}{\int_\lambda I(\lambda)F^2(\lambda)C(\lambda)\rho_s(\lambda)d\lambda} \\ \vdots \\ \dfrac{\int_\lambda I(\lambda)F^N(\lambda)C(\lambda)PPG(\lambda)d\lambda}{\int_\lambda I(\lambda)F^N(\lambda)C(\lambda)\rho_s(\lambda)d\lambda} \end{bmatrix} \right\| . \tag{4}$$

Here the PPG amplitude spectrum, PPG(λ), can be approximated by a linear mixture of the light absorption spectra from the two most common variants of the main chromophore in arterial blood, hemoglobin; oxygenated (HbO2) and reduced (Hb):

$$PPG(\lambda) \approx \epsilon_{Hb}(\lambda)c_{Hb} + \epsilon_{HbO_2}(\lambda)c_{HbO_2} = (1 - SaO_2)\epsilon_{Hb}(\lambda) + SaO_2\epsilon_{HbO_2}(\lambda)$$
$$= \epsilon_{Hb}(\lambda) + SaO_2[\epsilon_{HbO_2}(\lambda) - \epsilon_{Hb}(\lambda)], \tag{5}$$

where it is assumed that the optical path length differences are negligible for 600 < λ < 1000 nm and SaO2 ∈ [0, 1]. It is recognized that the wavelength-dependent effect of scattering could render this assumption invalid. When using two wavelengths, the ratio-of-ratios parameter R and the ratio of APBV parameter $\overrightarrow{P_{bv}}$ coincide. The wavelength selection may be based on three criteria: 1) the desire to measure oxygen saturation in darkness (λ > 700 nm) for clinical applications, 2) have a reasonable SpO2 contrast, and 3) wavelengths within the spectral sensitivity of the camera. The idea to use three instead of the common two wavelengths used in pulse-oximetry was motivated by the improved robustness of the SpO2 measurement by a factor of two. This can be explained by how motion affects the PPG waveforms when measured with a camera. Since motion-induced intensity variations are equal for all wavelengths, suppression of these artifacts is possible for the APBV method if the pulse signature $\overrightarrow{P_{bv}}$ is not equal to this motion signature, which can be described as a vector with equal weights.

It shall be noted that even if the pulse quality is very good, it does not always mean that the estimated SpO2 value is sufficiently reliable and can be trusted. This may particularly happen when unexpected blood-species (e.g. COHb) are available causing the SpO2 calibration curve to shift, i.e. causing a different signature vector to lead to the optimal pulse quality when using the PBV method or APBV method for pulse extraction.

It is critical in the above, that the PBV method assumes the relative pulsatilities in the wavelength channels are known, which is true if the desired vital sign information, e.g. the SpO2, were known. This however, in SpO2 monitoring, essentially is not the case since this is the parameter that is searched for. If the weights are chosen correctly, the correlation of the resulting pulse with the individual detection signals $C_n$ are exactly these relative strengths of the pulse in detection signals $C_n$. Now, if the vital sign information (e.g. the SpO2) is wrong or unknown, the result will be a pulse signal with a relatively poor SNR (i.e. a poor quality indicator).

The essence of the APBV method is to run a number of PBV methods in parallel with different PBV vector. The PBV method gives the cleanest pulse signal when the PBV vector, reflecting the relative pulsatilities in the different wavelength channels is accurate. Since this vector depends on the actual SpO2 value, testing the PBV method with different PBV vectors, corresponding to a range of SpO2 values, the SpO2 value results as the one corresponding to the PBV vector giving the pulse signal with the highest SNR.

Typically, the electromagnetic radiation used according to the present invention is in the range of 400 nm to 1000 nm for pulse, respiration and blood oxygen saturation measurement, particularly in the range of 620 nm to 920 nm. This particular range is most suitable for SpO2 measurement and is attractive for unobtrusive monitoring during sleep (darkness), but if pulse or respiratory signals are required, the visible part of the spectrum may allow a higher quality (i.e. NIR is not necessarily the preferred option in all cases).

The above described methods can be applied on detection signals that have been acquired using contactless sensors. By way of example, the present invention can be applied in the field of healthcare, e.g. unobtrusive remote patient monitoring, general surveillances, security monitoring and so-called lifestyle environments, such as fitness equipment or the like. Applications may include monitoring of oxygen saturation (pulse oximetry), pulse rate, blood pressure, cardiac output, respiration, blood perfusion variations, assessment of autonomic functions, and detection of peripheral vascular diseases. The present invention can e.g. be used for rapid and reliable pulse detection of a critical patient, for instance during automated CPR (cardiopulmonary resuscitation). The system can be used for monitoring of vital signs of neonates with very sensitive skin e.g. in NICUs and for patients with damaged (e.g. burnt) skin, but may also be more convenient than contact sensors as used in the general ward, and offer better solutions for motion robustness. Further application is in the automotive field. The present invention particularly solves issues with ambient-light robust multiple wavelength systems, offers a lower cost solution and eliminates privacy concerns.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

**Claims**

1. System for determining at least one vital sign of a subject, said system comprising:

   - an illumination unit (2) configured to illuminate a skin region of the subject, said illumination unit comprising a first radiation source (20) and a second radiation source (21), which are configured to emit differently modulated electromagnetic radiation in two or more wavelength channels and/or in two or more polarization channels,
   - a detection unit (4) configured to detect electromagnetic radiation reflected from the skin region of the subject and to generate detection signals,
   - a processing unit (11) configured to demodulate the detection signals corresponding to the modulation applied by the illumination unit to obtain demodulated detection signals, and
- a vital signs determination unit (5) configured to determine, per wavelength or polarization channel, a statistical measure from said demodulated detection signals and to extract a vital sign from said statistical measures.

2. System according to claim 1,
wherein the first and second radiation sources (20, 21) are configured to emit electromagnetic radiation at different wavelengths or wavelength ranges or mixtures of wavelengths.

3. System according to any one of the preceding claims,
wherein the first and second radiation sources (141, 142) are configured to emit electromagnetic radiation with different polarization.

4. System according to any one of the preceding claims,
wherein the illumination unit (2) is configured to apply modulation frequencies for modulating the emitted electromagnetic radiation in the range higher than 1 kHz, in particular in a frequency range from 10 kHz to 100 MHz.

5. System according to any one of the preceding claims,
wherein the first radiation source (20) is configured to apply a first modulation frequency for modulating the emitted electromagnetic radiation, which is at least 8 Hz apart from a second modulation frequency applied by the second radiation source (22) for modulating the emitted electromagnetic radiation.

6. System according to any one of the preceding claims,
wherein the statistical measure is indicative of at least one of a standard deviation, a variance, mean absolute difference, median absolute difference and/or an interquartile range.

7. System according to any one of the preceding claims,
wherein the detection unit (4) comprises a plurality of detection elements, in particular an array of photo diodes, a CCD array or a CMOS array.

8. System according to claim 3,
further comprising a polarization unit (10) configured to apply a polarization to the electromagnetic radiation reflected from the subject's skin region.

9. System according to claim 2,
further comprising an optical filter unit configured to filter the electromagnetic radiation reflected from the subject's skin region to allow only modulation frequencies used by the illumination unit to pass.

10. System according to any one of the preceding claims,
wherein the processing unit (11) is configured to apply synchronous demodulation on the detection signals to obtain the demodulated detection signals.

11. System according to any one of the preceding claims,
wherein the vital sign determination unit (5) is configured to determine a vital sign from the demodulated detection signals by linearly combining the demodulated detection signals by a weighted combination, wherein weights of said weighted combination are determined by blind signal separation, in particular by principal component analysis or independent component analysis, and by selecting a component channel of the combined detection signals according to a predetermined criterion.

12. System according to any one of the preceding claims,
wherein the vital sign determination unit (5) is configured to determine a vital sign from the demodulated detection signals by linearly combining the demodulated detection signals by a weighted combination using weights resulting in a pulse signal for which the products with the original detection signals equals the relative pulsatilities as represented by the respective signature vector, a signature vector providing an expected relative strength of the detection signal in the original detection signals.

13. System according to any one of the preceding claims,
wherein at least one of the first and second radiation sources (20, 21) is configured to emit a controllable narrow radiation beam.

14. System according to any one of the preceding claims,
    wherein the vital signs determination unit (5) is configured to determine, per wavelength or polarization channel, a central tendency metric and a dispersion metric as statistical measures from said demodulated detection signals.

15. Method for determining at least one vital sign of a subject, said method comprising:

    - illuminating a skin region of the subject by an illumination unit, said illumination unit comprising a first radiation source (20) and a second radiation source (21), which are configured to emit differently modulated electromagnetic radiation in two or more wavelength channels and/or in two or more polarization channels,
    - detecting electromagnetic radiation reflected from the skin region of the subject and to generate detection signals,
    - demodulating the detection signals corresponding to the modulation applied by the illumination unit to obtain demodulated detection signals,
    - determining a statistical measure, per wavelength channel or per polarization channel, from said demodulated detection signals, and
    - extracting a vital signal from said statistical measures.

FIG.1

FIG.2

FIG.3

FIG.4

Europäisches
Patentamt

European
Patent Office

Office européen
des brevets

## EUROPEAN SEARCH REPORT

Application Number

EP 18 17 7303

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/243622 A1 (CROWE JOHN [GB] ET AL) 28 August 2014 (2014-08-28) | 1-15 | INV. A61B5/00 A61B5/024 A61B5/145 A61B5/1455 |
| Y | * paragraphs [0008], [0009], [0013], [0021], [0060], [0061], [0073] - [0093], [0153]; claims 1,7; figures 3-5b * | 1-15 | |
| X | WO 2016/166651 A1 (KONINKLIJKE PHILIPS NV [NL]) 20 October 2016 (2016-10-20) <br><br> * paragraphs [0001], [0003], [0008], [0011], [0015], [0017], [0018], [0038], [0040], [0046], [0048] - [0050], [0054], [0056], [0058]; claims 1,2,4,6,9; figure 4 * | 1,2,4,5,7,9,13,15 | |
| X | WO 2011/117780 A1 (KONINKL PHILIPS ELECTRONICS NV [NL]; VEEN JEROEN [NL]; JANSEN THEODORU) 29 September 2011 (2011-09-29) <br> * page 6, lines 29-34; figures 1,2 * | 1,2,10,13,15 | TECHNICAL FIELDS SEARCHED (IPC) A61B |
| Y | MARK VAN GASTEL ET AL: "Camera-based pulse-oximetry - validated risks and opportunities from theoretical analysis", BIOMEDICAL OPTICS EXPRESS, vol. 9, no. 1, 1 January 2018 (2018-01-01) , page 102, XP55498320, United States ISSN: 2156-7085, DOI: 10.1364/BOE.9.000102 * 2.2.2 APBV * | 1-15 | |

-/--

The present search report has been drawn up for all claims

| Place of search | Date of completion of the search | Examiner |
|---|---|---|
| The Hague | 26 November 2018 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 18 17 7303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE HAAN G ET AL: "Improved motion robustness of remote-PPG by using the blood volume pulse signature", PHYSIOLOGICAL MEASUREMENT, INSTITUTE OF PHYSICS PUBLISHING, BRISTOL, GB, vol. 35, no. 9, 27 August 2014 (2014-08-27), pages 1913-1926, XP020269523, ISSN: 0967-3334, DOI: 10.1088/0967-3334/35/9/1913 [retrieved on 2014-08-27] * the whole document * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 26 November 2018 | Lommel, André |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

**EP 3 581 091 A1**

## ANNEX TO THE EUROPEAN SEARCH REPORT
## ON EUROPEAN PATENT APPLICATION NO.

EP 18 17 7303

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2018

| Patent document cited in search report | | | Publication date | Patent family member(s) | | | Publication date |
|---|---|---|---|---|---|---|---|
| US 2014243622 | A1 | | 28-08-2014 | AU | 2007242649 | A1 | 01-11-2007 |
| | | | | CA | 2649187 | A1 | 01-11-2007 |
| | | | | CN | 101484065 | A | 15-07-2009 |
| | | | | EP | 2004038 | A2 | 24-12-2008 |
| | | | | ES | 2556648 | T3 | 19-01-2016 |
| | | | | JP | 5270533 | B2 | 21-08-2013 |
| | | | | JP | 2009533121 | A | 17-09-2009 |
| | | | | US | 2009306487 | A1 | 10-12-2009 |
| | | | | US | 2014243622 | A1 | 28-08-2014 |
| | | | | WO | 2007122375 | A2 | 01-11-2007 |
| | | | | ZA | 200809559 | B | 25-11-2009 |
| WO 2016166651 | A1 | | 20-10-2016 | NONE | | | |
| WO 2011117780 | A1 | | 29-09-2011 | CN | 102811663 | A | 05-12-2012 |
| | | | | EP | 2549926 | A1 | 30-01-2013 |
| | | | | JP | 6184318 | B2 | 23-08-2017 |
| | | | | JP | 2013530728 | A | 01-08-2013 |
| | | | | WO | 2011117780 | A1 | 29-09-2011 |

EPO FORM P0459

For more details about this annex : see Official Journal of the European Patent Office, No. 12/82

20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013271591 A1

**Non-patent literature cited in the description**

- **G. DE HAAN ; A. VAN LEEST.** Improved motion robustness of remote-PPG by using the blood volume pulse signature. *Physiol. Meas.,* 2014, vol. 35, 1913

- **M. VAN GASTEL ; S. STUIJK ; G. DE HAAN.** New principle for measuring arterial blood oxygenation, enabling motion-robust remote monitoring. *Nature Scientific Reports,* November 2016